# EUROPEAN PATENT SPECIFICATION

(11) **EP 3 558 264 B1**
(45) Date of publication and mention of the grant of the patent: **17.07.2024**
(21) Application number: 17837847.7
(22) Date of filing: 22.12.2017
(51) Int. Cl.: A61K 9/00

(54) **OPHTALMOLOGIC PREPARATION**
OPHTHALMOLOGISCHE ZUBEREITUNG
PRÉPARATION OPHTALMOLOGIQUE

(30) Priority: 23.12.2016 CZ 20160827
(43) Date of publication of application: 30.10.2019
(73) Proprietor: Contipro A.S., 561 02 Dolní Dobrouc (CZ)
(72) Inventor: KNOTKOVA, Katerina, 56501 Chocen (CZ); KOTZIANOVA, Adela, 74705 Opava (CZ); VELEBNY, Vladimir, 56401 Zamberk (CZ)
(74) Representative: Dvorakova, Martina
(86) International application number: PCT/CZ2017/050066
(87) International publication number: WO 2018/113805

(56) References cited:
- EP-A1- 3 108 874
- US-A1- 2011 229 551
- US-A1- 2016 312 385

## Description

### Field of the invention

The present invention relates to an ophthalmologic preparation in the form of solid fibrous carrier comprising water soluble nano-fibers containing hyaluronic acid or a pharmaceutically acceptable salt thereof and/or at least one derivate thereof, further containing water soluble synthetic polymer and an active agent.

### Background of the invention

In the field of pharmaceutical applications, the application of medical agents through specific sites and specific routes is essential for obtaining desired effect. Also critical is the dosage regimen that ensures the administration of the right dose of medical agents. Because of the anatomical and physiological uniqueness of the eye, the distribution of drugs for treatment of ophthalmologic diseases and disorders has been challenge for long time.

Currently, in fact all ophthalmologic drugs are produced in the form of eye drops despite it being impossible to ensure sufficient duration of the therapeutic agent on the cornea. Moreover, the absorption and effect of the therapeutic agent is influenced by many other factors such as blood circulation in the conjunctiva, its clearance with lymph and, above all, its dilution with tears and binding of the drug to proteins contained in tears. Due to above mentioned risks, there still exists the need of alternative form of ophthalmologic drugs administration, that enables to overcome at least some of the issues of commonly available forms (Nagarsenker, M., et al. (1999). "Preparation and evaluation of liposomal formulations of tropicamide for ocular delivery." International Journal of Pharmaceutics 190(1): 63-71). Eye drops made up about 90 % of drug forms for ocular applications from several reasons, those being costs, good acceptability by patients and simple preparation (Le Bourlais, C., et al. (1998). "Ophthalmic drug delivery systems - recent advances." Progress in retinal and eye research 17(1): 33-58). Despite all these advantages, the eye drops are highly ineffective means, whereas only 5% of drug penetrates through the cornea (Lang, J. C. (1995). "Ocular drug delivery conventional ocular formulations." Advanced Drug Delivery Reviews 16(1): 39-43).

Following the drug application in the form of solution, the drug is diluted with tears. The time of drug contact with the eye is short (1-2 minutes) because the eye produces 0.5-2.2 µl of lacrimal fluid per minute. Moreover, the blinking frequency (12 × per minute) increases lacrimal fluid clearance and thus increases the clearance of eye drops (drug) from the site of application (Ahmed and Patton 1987, "Disposition of timolol and inulin in the rabbit eye following corneal versus non-corneal absorption." International Journal of Pharmaceutics 38(1-3): 9-21). Such drug removal is solved by the increase of the dose thereof. The eye is thus exposed to the risk of unpredictably high dose of drug, but in some cases, none might be absorbed. In the same time, it is more difficult to determine the period between individual drug administrations.

These data imply that there still exists the need to improve the properties and drug regimens of medical agents for ocular applications, despite the fact, that the eye drops make up 90 % of drug forms. The ideal system for drug dosage into the eye should not irritate the eye, should not cause foggy sight or some other kinds of irritation or burning and moreover should be in ideal case applied at maximum 2 × a day (Van Ooteghem, M. (1995). Préparations ophtaliniques, Technique & Documentation-Lavoisier.).

Polymer gels that improve eye reaction and decrease side effects such as system reaction to topically administered ocular drug are one such solution. The gels enable longer drug contact with the tissue and prolong the period of drug absorption and reduce the dosage frequency. (Le Bourlais, Acar et al. 1998).

Due to its regenerative and moisturizing effects, the hyaluronic acid has been long part of ocular medicine. Eye surgeons inject hyaluronic acid into the eye to ensure that the eye ball retains its shape during the operation (Rah, M. J. (2011). "A review of hyaluronan and its ophthalmic applications." Optometry-Journal of the American Optometric Association 82(1): 38-43). Hyaluronic acid is also the part of some solutions for eye lenses and eye drops that display prominent moisturizing, tranquilizing, regenerative and protective effect (Hyal-Drop^{®}, Hylo-gel^{®}, Hylo-care^{®}).

Further possibilities that have been studied and applied by many research groups are contact lenses with the content of drug itself or in the form of liposomes, nanomaterials etc. There exist several methods of drug incorporation into contact lenses. Basic and a simplest method is form of impregnation where the lens is soaked in the drug solution and then is applied into the eye (Hehl, Beck et al. "Improved penetration of aminoglycosides and fluoroquinolones into the aqueous humour of patients by means of Acuvue contact lenses." European journal of clinical pharmacology 55(4): 317-323). However, this approach brings about problems with dosage because the drug content in the lens is only partially controllable and further limited. The newest research works with drug in the form of nanomaterials (encapsulated drug), that are built into the lenses. Such nanomaterial is then produced from a material, from which the drug may diffuse and then migrate from the lens towards eye tissue (see EP 15324202). The size of particles with the drug content 1-5% does not excess 50 nm. Nevertheless, the application of lenses into the eye is difficult, may lead to eye irritation, inconvenient feelings such as tingling, burning in the eye. Also, the eye infection risk increases.

Contact lenses made of nano-fibers/fibers or containing nano-fibrous or fibrous mesh have been in the center of attention for past decades. Nano-fibers appear, due to their properties, as suitable material for targeted drug distribution and controlled release (Valmikinathan, C. M., et al. (2009). "Polycaprolactone and Bovine Serum Albumin Based Nanofibers for Controlled Release of Nerve Growth Factor." Biomacromolecules 10(5): 1084-1089) and for formation of scaffolds as cell carriers (Kim, K., et al. "Control of degradation rate and hydrophilicity in electrospun non-woven poly(D,L-lactide) nanofiber scaffolds for biomedical applications." Biomaterials 24(27): 4977-4985, 2003) with controlled material hydrophobicity. The combination of nano-fibers and contact lens then represents better and simplified drug application, wherein the nano-fiber layer is prepared from synthetic polymers containing the drug, whereas the drug is incorporated only after the nano-fiber preparation. The microparticles are then produced from the nano-fibrous layer and incorporated into the lenses. Further option is to apply directly the nano-fibrous layer without the necessity to produce lenses. Similar approach is described in the patent document WO 2010/120489, wherein the drug carrier is produced as a nano-fibrous layer from synthetic insoluble polymer, whereas the drug is incorporated into the nano-fibers by immersing them into the drug solution. Polymer in nano-fibrous layer is then meshed and the layer is coated with another layer of synthetic insoluble polymer, that being again meshed. This layer then forms the protective layer for nano-fibers and retards drug release. Nevertheless, in view of the fact, that it does not relates to a water-soluble material, inconvenient eye irritation may occur as well as foggy seeing. Moreover, residuals of surface active agents, meshing agents used for the layer preparation may remain here.

The patent document US 20110229551 relates to nano-fibers as direct drug carrier that is applied into body fluids, where the body fluid is blood or other body fluids. The carrier is in this case made of nano-fibers of biodegradable synthetic polymers such as PLGA and PLLA, that however are not water-soluble, and their time of degradation thus prolongs. In the case of eye application, it causes longer persistence within months, if the material is not removed. Other nano-fibrous carriers for controlled release are prepared from mucoadhesive polymers such as polyvinyl alcohol, alginate and in the case of other polymers with the addition of other agents such as plastifiers, inorganic salts, non-degradable polymers and drugs (Shivani, et al. (2014) IN 201400204I1. Topical nano-fibrous ocular implants). Another patent document WO2013/171764 describes the product for eye applications that contains so called Greek hay, that forms the basis of this preparation. Other agents are then added to this preparation, such as superficially active agents, stabilizers, plastifiers and before all fillers (see WO 2013171764). The additions of plastifiers, surfactants and other agents that are not substances of the body, represent a burden for the organism and it may cause irritation or allergic reaction.

Nano-fibers of polyvinyl alcohol, polyethylene oxide and hydroxy propyl methyl cellulose, eventually of the mixture thereof were used also as carriers with the drug addition, which solves patent document US2013/136784. Disadvantage is the use of hydroxy propyl methyl cellulose, that acts in its essence as a filling in contrast to our use of hyaluronic acid, that acts here also as a moisturizer and is natural for the eye. Apart from that, it is the purpose of this patent document to produce nano-fibrous layer that serves in its essence as the precursor to produce a tampon with the drug content. In contrast to other agents intended for eye applications, this preparation is primarily determined as vaginal tampon with spermicidal effect.

Patent document US2016/312385 describes preparations comprising photocured nanofibers made of photocurable hyaluronic acid derivate. Such preparations are not soluble in water. Such preparation can be used in cosmetics or medicine, preferably as a wound care device, patches.

### Summary of the invention

The object of the present invention is to develop the ophthalmologic preparation that would overcome above stated drawbacks and enable an application with a precise dosage of the active agent and whose application would be very simple and the drug release would occur only once and only at its dissolution at the contact with the eye, which would secure the release of proper amount of one or more active agents.

Such assumptions are fulfilled with the ophthalmologic preparation containing hyaluronic acid, characterized in that it is in the form of the solid fibrous carrier that comprises water-soluble nano-fibers containing hyaluronic acid, or a pharmaceutically acceptable salt thereof selected from the group containing Na⁺, K⁺, Mg²⁺ or Ca²⁺, preferably Na⁺,
and/or at least one derivative of hyaluronic acid of general formula I, wherein 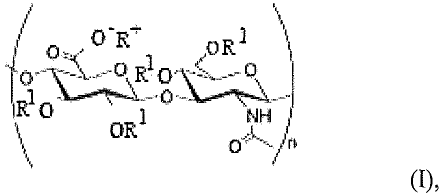
wherein R is H⁺ or pharmaceutically acceptable salt selected from the group containing Na⁺, K⁺, Mg²⁺ or Ca²⁺, preferably Na⁺,
R¹ is H or -C(=O)CₓH_{y} or furanyl, wherein x is integer in the range from 3 to 21 and y is integer in the range from 7 to 43 and CₓH_{y} is linear or branched, saturated or unsaturated chain C₃-C₂₁, whereas at least in one repeating unit is one or more R¹-C(=O)CₓH_{y} or furanyl, and where n is in the range 12 to 4000;
further, at least one water-soluble synthetic polymer selected from the group containing polyethylene oxide or polyvinyl alcohol, the content of the synthetic polymer or the mixture thereof being in the range 10 to 90 wt.% in the dry mass of nano-fibers contained in the ophthalmologic preparation;
and at least one active agent, preferably one ophthalmologically active agent.

Molecular mass of hyaluronan or derivative thereof is in the range 10 000 to 300 000 g/mol, preferably 15 000 to 150 000 g/mol, more preferably 40 000 to 115 000 g/mol. The degree of hyaluronan derivatives substitution is in the range from 2 to 75%, preferably from 2.5 to 66%.

According to preferable embodiment of the present invention, the derivative of hyaluronic acid is selected from the group containing palmitoyl hyaluronan, caproyl hyaluronan, oleyl hyaluronan, butanoyl hyaluronan, linoleoyl hyaluronan, lauryl hyaluronan or furanyl hyaluronan.

Further, the active agent is selected from the group containing antiallergic, antibiotic, antimycotic, antineoplastic, anti-inflammatory, antivirotic, antiglaucoma, antiseptic or diagnostic agents. Examples of ophthalmologically active agents without limitation are: tetryzoline, levocabastine, gentamycin, ofloxacin, neomycin, clotrimazole, terbinafine, nystatin, doxorubicin, prednisolone, dexamethasone, diclofenac, ketorolac, acyclovir, latanoprost, dorzolamide, bimatoprost, tafluprost, travoprost, brinzolamide, carbetopendecium, potassium iodide, octenidine.

If the active agent, preferably the ophthalmologically active agent, is hydrophobic, it is contained in the nano-fibers in the form of micelles.

The content of active agent, preferably ophthalmologically active agent, is 0.01 to 50 wt.%, preferably 0.1 to 50 wt.% in the dry mass of nano-fibers contained in ophthalmologic preparation according to the present invention.

The content of hyaluronic acid or a pharmaceutically acceptable salt and/or derivative thereof according to general formula I is in the range 5 to 90 wt.%, preferably 10 to 90 wt.%, more preferably 50 to 80 wt.% in the dry mass of nano-fibers contained in the ophthalmologic preparation according to the invention. Mass ratio in the mixture of hyaluronic acid or a salt and derivative of hyaluronic acid is 1/9 to 9/1, preferably 3/7 to 7/3.

The content of synthetic polymer or the mixture thereof is in the range 10 to 90 wt.%, preferably 20 to 50 wt.% in the dry mass of nano-fibers contained in the ophthalmologic preparation according to the present invention. Mass ratio of polyethylene oxide and polyvinyl alcohol in the mixture is in the range of 19/1 to 3/17.

Nano-fibers contained in ophthalmologic preparation according to the invention have diameter in the range of 50 to 1 000 nm, preferably 50 to 500 nm, more preferably 50 to 200 nm.

The ophthalmologic preparation according to the invention is in the form of layer, its areal weight being in the range of 1 to 100 g/m², preferably 1 to 20 g/m², more preferably 3 to 17 g/m2.

The ophthalmologic preparation according to the invention is preferably equipped with a holder for manipulation during its application into the eye.

The ophthalmologic preparation according to the invention is suitable for use in treating or prevention of ophthalmologic states, preferably ophthalmologic diseases and disorders chosen from the group comprising glaucoma, dry-eye syndrome, macular degeneration, infection, inflammation or allergy.

### Detailed description of the invention

Hyaluronan is among glycosaminoglycans unique body non-sulfate. It is unique in that it originates in a plasma membrane instead of Golgi apparatus and its molecular mass may reach millions Da. Hyaluronan as one of the main components of extracellular matrix essentially contributes to cellular proliferation and migration. Hyaluronic acid is a body's own acid and it acts in the preparation according to the present invention particularly as a moisturizer.

The present invention is based on long studied knowledge that the eye medication may be easily administered without the risk of its spilling or overdosing thanks to a solid application form, specifically in the form of solid fibrous carrier, thus without the need of liquid form. These nano-fibers, at the contact with aqueous environment such as tear fluid, dissolve very rapidly and release the active agents, that had been incorporated into the solid fibrous carrier during its production. The application of this solid fibrous carrier, that is usually in the shape of a band, is very simple and thanks to very fast dissolving of nano-fibrous carrier also surprisingly comfortable. These properties make thus the present solid fibrous carrier ideal for delivery of specific doses of mentioned active agents into the eye. Since the solid fibrous carrier dissolves only in the case of its contact with wet environment of the eye, no preliminary release of active agents, preferably medical agents, occurs and the proper dosage is such secured.

As was described above, the present invention is first of all ophthalmologic preparation for the treatment of ophthalmologic diseases and disorders, whereas this preparation comprises solid carrier in the form of water-soluble nano-fibers, resp. fibrous layer that contains at least one active agent. This solid fibrous carrier is soluble at the contact with the eye.

The solid fibrous carrier represents pharmaceutically acceptable composition that delivers the active agent at the place of cure.

Within the scope of present invention, the carrier is a solid composition at standard pressure and temperature (20 °C, 101.3 kPa) that comprises water-soluble nanofibers in the form of the fibrous layer. This fibrous layer is water soluble and thus it dissolves easily in aqueous environment. The nano-fibers preferably dissolve instantly at their contact with wet environment, preferably tear fluid.

The fibrous layer is a structure made of individual nano-fibers in random or oriented organization. Fibrous layers are made of one of stated technologies known under the names of electrospinning or electro-blowing, optionally melt-blowing. Processes of preparation of nano-fibers have been numerously published (Ramakrishna, S., et al. (2005). An introduction to electrospinning and nanofibers, World Scientific), wherein the materials are not limited by their solubility or biodegradability.

The advantage of the ophthalmologic preparation according to the present invention in the form of solid fibrous carrier is low diameter of nano-fibers, large specific surface and high number of small pores between fibers. These properties lead to the increase in nano-fibers reactivity and thus to fast release and shortening of diffusion routes for incorporated medical agents. The solid fibrous carrier of the ophthalmologic preparation according to the invention is prepared from the mixture of hyaluronan and at least one water-soluble synthetic polymer, for example, polyethylene oxide or polyvinyl alcohol. These properties enable fast disintegration of the solid fibrous carrier. For achieving desired parameters of dissolution kinetics at contact with negligible amount of liquid, such as is the contact with wet cornea surface, nano-fibers have their diameters in the range, as was stated above. Specific areal mass of fibrous layers according to the invention varies in the range of 1 to 100 g/m², preferably however in the range of 1 to 20 g/m², more preferably in the range of 3 to 17 g/m². In preferred embodiments of present invention, the profile of active compound release may be adjusted by changing the structure of fibrous layer (i.e. by the fiber diameter). Nano-fiber diameter may be influenced using HA derivatives or mixtures thereof with native HA, the use of the derivative also determines the dissolution rate and eventual layer hydrophobicity. In the case of furanyl derivative of HA, the time of meshing plays an important role. UV irradiation is used for its meshing. The time of irradiation is preferably about 10 minutes.

Although the solid fibrous carrier might be prepared in various shapes and dimensions varying according to specific application, its typical shape is the band, whose dimension is suitable for ocular application. Such band is roughly of rectangular shape with following dimensions - length from 5 to 100 mm, preferably 10 to 60 mm, width 1 to 20 mm, preferably 1 to 10 mm and thickness of fibrous layer is from 0.010 to 2 mm, preferably 0.01 to 1 mm. Medical agent remains in solid fibrous carried until it is dissolved at its contact with aqueous solution, for example with tear fluid in the eye.

The solid carrier comprising water-soluble nano-fibers in the form of the fibrous layer has the advantage that it dissolves very quickly, most often within seconds, and therefore this embodiment of present invention is very advantageous and unique. Eventual uncomfortable feeling during the application of the preparation stops very quickly. Initial feeling however assures the patient about proper application of the ophthalmologic preparation. Moreover, the advantage of this preparation is that it provides explicit feedback, whether the medical agent was or was not applied into the eye. If the application occurs, the fibrous carrier dissolves. For example, if the patient with tremor misses the eye during the application, the solid fibrous carrier remains in solid and thus easily determinable form and the patient can repeat the application. Specific and very clearly defined dose of medical agent may thus be delivered into the eye.

The solid fibrous carrier in various embodiments of the preparation according to the present invention contains water soluble nano-fibers with the content of native hyaluronic acid or a pharmaceutically acceptable salt thereof and/or at least one pharmaceutically acceptable derivative of hyaluronic acid and further it contains the synthetic polymer and the active agent. Hyaluronan esters of C₄-C₂₂ fat acids and furanyl derivative of hyaluronic acid belong among used derivatives of hyaluronic acid, preferably palmitoyl hyaluronan, caproyl hyaluronan, lauryl hyaluronan, oleyl hyaluronan, butanoyl hyaluronan, linoleyoyl hyaluronan and their pharmaceutically acceptable salts of alkali metals, preferably sodium salt.

These derivatives are especially preferable for the preparation of solid fibrous carriers of biologically active agents. The solubility of the solid carrier in the form of fibrous layer may be optimized for given application using the suitable type of derivative of hyaluronic acid of the general formula I above, and as well as by its concentration in fibrous carrier. Properties and parameters of the nano-fibers, and also of fibrous layers, may be adjusted by proper choice of hyaluronic acid and/or the derivative thereof, eventually more derivatives. Moreover, the choice of the type of hyaluronic acid may change the profile of medical agent release and it may be prepared the best material for given application.

The pharmaceutical preparation according to the present invention for the use in ophthalmology with high content of hyaluronic acid consists of native hyaluronic acid or pharmaceutically acceptable salt thereof and/or pharmaceutically acceptable derivative of hyaluronic acid of general formula I above and further of synthetic polymer (for example of polyethylene oxide, polyvinyl alcohol and mixtures thereof) wherein hyaluronan concentration ranges from 5 to 90 wt.%, preferably 10 to 90 wt.%, more preferably 50 to 80 wt.%, most preferably 60 to 80 wt.%. The water soluble synthetic polymer for electrospinning is for example polyethylene oxide, polyvinyl alcohol or mixtures thereof, that are added due to the preparation of the fibrous layer. The amount of the water soluble synthetic polymer in nano-fibers ranges from 10 to 95 wt.%, preferably 10 to 90 wt.%, more preferably 10 to 50 wt.%, most preferably 20 to 40 wt.%. The derivative of hyaluronic acid may completely replace native hyaluronan and thus its concentration ranges from 5 to 90 wt.%, preferably 10 to 90 wt.%, more preferably 50 to 80 wt.%, most preferably 60 to 80 wt.% or may partially replace hyaluronan such that the concentration of both (hyaluronic acid and the derivative thereof) ranges from 5 to 90 wt.%, preferably 10 to 90 wt.%, more preferably 50 to 80 wt.%, most preferably 60 to 80 wt.%. The amount of active agent, preferably ophthalmologically active agent, may range from 0.01 to 50 wt.%, preferably 0.1 to 50 wt.%. Its concentration than depends on the specific drug. Properties and parameters of fibrous layers may be adjusted by the right choice of hyaluronic acid and/or the derivative thereof, eventually more derivatives, while various rates of solubility at the contact with the aqueous solution, solubility of polar and/or hydrophobic medical agents may be achieved.

In tested embodiments according to the present invention, the solid fibrous carrier is dissolved within 0.05 to 10 seconds, preferably 0.1 to 1 seconds, more preferably 0.05 to 0.5 second. Thanks to this fast disintegration of solid fibrous carrier, the ophthalmologic preparation according to the invention may be thus easily administered by the untrained patient itself. The application is simplified especially for patients suffering of tremor a/or for elderly patients, for whom is the administration of common eye medicaments such as eye drops, often complicated. "Complete dissolution" of the solid fibrous carrier marks the state when the disintegration of the solid fibrous carrier and thus also the dissolution of water soluble nano-fibers occurs so that individual molecules of nano-fibers are hydrated. In the case of water soluble nano-fibers according to the invention containing hyaluronic acid and/or at least one derivative thereof and nano-fibers prepared thereof, the disintegration to individual chains of hyaluronic acid and/or the derivatives thereof occurs. The complete dissolution thus means that 50% to 100% or preferably 70% to 100% and more preferably 90% to 100% of water soluble nano-fibers is disintegrated to individual molecules. Water soluble nano-fibers of hyaluronan or the derivative thereof or the mixture thereof is so completely dissolved immediately upon its contact with the aqueous medium such as e. g. tear fluid, at the latest within 10 seconds.

In some embodiments of the present invention, the solid fibrous carrier in the form of the fibrous layer is completely dissolved immediately upon its contact with aqueous medium such as tear fluid, i.e. the time necessary for disintegration is to 10 seconds, more preferably to 1 second, most preferably from 0.05 to 0.5 second.

Such embodiments of the present invention are especially advantageous, as they enable in fact immediate application of medical agents into the eye of patients and it cause no pain, irritation or uncomfortable feeling. In other words, the administration of the medical agent occurs once the patient obtaining the preparation of present invention blinks. This is especially advantageous in the cases, when the patient obtaining the treatment is not able of proper application of conventional eye drops, e. g. lie or she is not able to press the bottle properly or suffers with tremor (patients with insufficient strength of hands and/or with neurodegenerative disorder e. g. Parkinson's disease).

As it was stated above, the present invention is, in the first place, the ophthalmologic preparation for the treatment of ophthalmologic diseases and disorders, whereas this preparation is in the form of the solid carrier in the form of the fibrous layer that contains at least one medical agent, whereas this solid fibrous carrier is soluble at the contact with the eye.

Active agents, preferably ophthalmologically active agents, are in the ophthalmologic preparation according to the present invention contained in the efficient amount that is defined as the amount of active agent that is sufficient to achieve desired local or systemic effect and efficiency at acceptable rate between the risk and the gain.

At least one the active agent is applied into the nano-fibers, resp. fibrous layer during its production. The drug application into the fibrous layer is ensured by addition of the active agent in the form of powder (spray) or a solution into the mixture/solution intended for the preparation of the solid fibrous carrier. Then the active agent is contained in whole capacity of fiber. The content of the active agent is uniform in fibers, thus also in the fibrous carrier. Moreover, it is possible to prepare the fibrous carrier simply in one step of spinning.

Solid hydrophobic drugs are added in the form of micelles, wherein the body of a micelle is formed by the derivative of hyaluronan according to the present invention and the drug is encapsulated inside the micelle. Liquid hydrophobic drugs may be added in their own form making an emulsion that is spun. To form micelles the emulsion must contain at least one derivative of hyaluronan according to the invention and the hydrophobic ophthalmologic active agent. The hydrophobic ophthalmologic active agent is a nonpolar agent, insoluble in water.

Moreover, the advantage of the ophthalmologic preparation is that it provides the clear feedback, whether the medical agent was or was not applied into the eye. Once the application occurs, the solid fibrous carrier dissolves. For example, in the case when the patient with tremor misses the eye during the application, the solid fibrous carrier remains in solid and thus easily recognizable form and the patient may repeat the application. Specific and very clearly defined dose of the medical agent may be thus administered into the eye. Present invention comprises the solid fibrous carrier containing one dose of the ophthalmologic preparation according to the present invention.

The ophthalmologic preparation according to the invention may thus contain additional pharmaceutically acceptable excipients selected from the group containing antioxidants, vitamins or inorganic salts.

Excipients may be those that are solid in their standard form and at room temperature, e.g. antioxidants, vitamins and inorganic salts or the combinations thereof. Among these inorganic salts belong potassium chloride, sodium chloride, calcium chloride, magnesium chloride, however, the use is not limited to these inorganic salts only. Antioxidants may be tocopherol, tocotrienol, tocomonoenol or gamma-tocopherol (MDT) in limited amount (to 5%). Antioxidants may be selected from the group containing alpha-, beta-, gamma-, delta-tocopherol, alpha-, beta-, gamma-, delta-tocotrienol, alpha-, beta-, gamma-, delta-tocomonoenol, alpha-, beta-, gamma-, delta-MDT and the mixtures thereof.

The ophthalmologic preparation according to the invention may achieve, upon dissolution, pH in the range 6 to 8, preferably around 7.

The present ophthalmologic preparation does not contain any preservatives. Thus, it is possible to avoid eventual irritation that could cause some preservatives. Long-term stability of the preparation is ensured by the solid fibrous carrier itself, whose method of preparation enables to avoid preservatives. The ophthalmologic preparation is preferably packed sterile, i.e. in sterile packets or blisters. The sterilization is performed using ethylene oxide (ETO) or gamma irradiation.

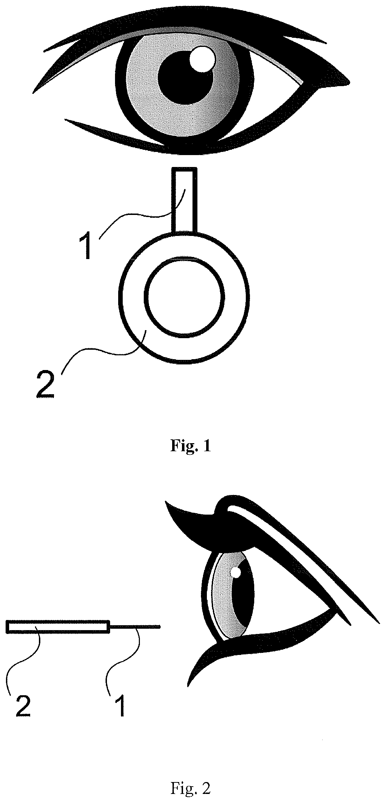

For easier manipulation, the ophthalmologic preparation according to the invention may be attached into a holder that is adapted for the ocular application of the ophthalmologic preparation. The ophthalmologic preparation, preferably in the shape of the band, is attached into the holder using usual procedures. The ophthalmologic preparation is inserted into the holder so that the part of the band containing desired amount of drug remains free (Fig. 1). This is preferable especially for patients with tremor and/or for elder patients. Further, it also enables the preparation of the ophthalmologic preparation so small that the uncomfortable feeling during the application is also minimalized and thus also the time of dissolution of solid fibrous carrier.

The present invention comprises the ophthalmologic preparation, whereas some embodiments are then particularized for treatment and prevention of ophthalmologic states such as ophthalmologic diseases and disorders. Some of preferable embodiments of the ophthalmologic preparation according to the present invention may be used in the field of ophthalmologic diseases or disorders such as glaucoma, syndrome of dry eye, macular degeneration, infection, inflammation and allergy.

### Definition of terms

All wt.% are relative to nano-fiber dry mass.

The term "ophthalmologic preparation" refers to the preparation prepared in a method so that it is suitable for the administration to people e.g. the method of preparation suits the conditions of Good manufacturing practices (GMP). The preparation is in solid form and it may have the shape of the band. Typically, the preparation is produced from the solid fibrous carrier that comprises nano-fibers with the content of HA or pharmaceutically acceptable salts thereof and/or at least one derivative of HA of general formula I above and further, it contains water soluble synthetic polymer and at least one active agent and it may contain also additional excipients.

The term "dissolved" means the result of the process of disintegration of the solid fibrous carrier in the solvent to the extent that it is no longer registered by the patient to whom it was administered or that the patient does not feel it. This process comprises separation and/or dissolution of water soluble fibrous layer so that its structural integrity (inner structure) is disrupt and it is no longer perceived by the patient as a (solid) foreign object.

The terms "hyaluronan", "hyaluronic acid" and "HA" are interchangeable and refer to anionic, non-sulfate, glycosaminoglycan distributed especially by connective, epithelium and nervous tissue.

The term "active agent" refers herein to an agent that is to be administered, the agent able to achieve the desired effect or the intended action. Such agent comprises, without limitation, ophthalmologically active agent i.e. medical agent but also diagnostical agents and mixtures thereof. The desired effect or intended action may comprise, without limitation, prophylactic effect to organism, prevention of undesired effects (e.g. infection) and alleviation of the course of the disease (e.g. alleviation of pain or infection caused by the disease) and/or alleviation or complete elimination of the disease from the organism). The effect may be local or systemic. Treated organisms are mammals, predominantly humans.

The term "water soluble synthetic polymer" means the biocompatible water soluble synthetic polymer that may be independently electrostatically spun. It is pharmacologically acceptable, and it does not cause any undesired local or systemic toxicity.

The term "ophthalmologic state" refers to the state, disease or disorder of the eye from uncomfortable feeling registered by the patient without disease up to serious incurable eye disease including adjacent tissues, fluids and eye muscles, where the disease is defined as a state causing pain, dysfunction, suffering, social problems to affected person or similar problems to those who are in contact with said person including injury, defects, disorders, syndromes, infections and unique symptoms.

### Brief description of the drawings

The present invention will be better understood with the submission of detailed description along with the attached drawings.
Fig. 1 shows the embodiment wherein a place intended for its attaching and manipulation is part of the solid fibrous carrier. Solid fibrous carrier (1) that is attached to the holder (2) that may be used once or repeatedly is displayed in detail.
Fig. 2 schematically displays the application of the solid fibrous carrier using the holder (2) into the patient's eye without him or her having to touch the eye. After the contact with tear fluid occurs complete dissolution of solid fibrous carrier and active agents release.

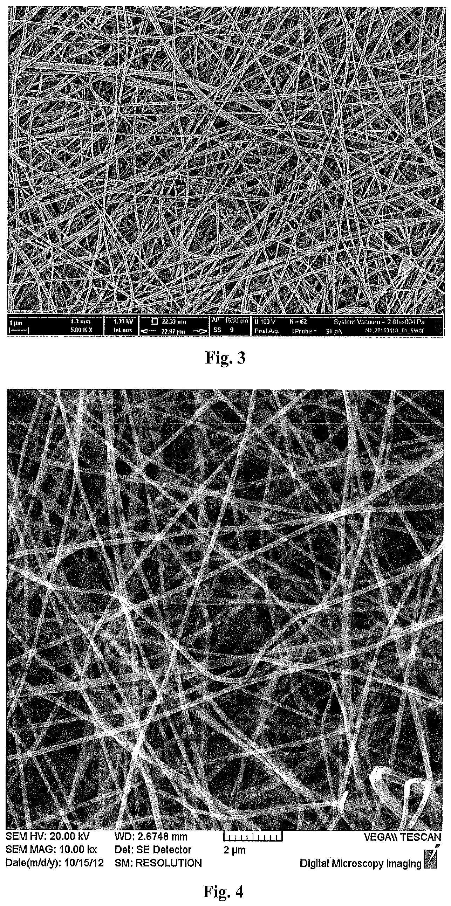

Fig. 3 displays the fibrous layer of hyaluronic acid (molecular mass 81 000 g/mol; 79.6 wt.%) and polyethylene oxide (molecular mass 400 000 g/mol; 20 wt.%) containing octenidine with concentration 0.4 wt.%. Fiber diameter 100 ± 30 nm.
Fig. 4 shows fibrous layer of hyaluronic acid (molecular mass 76 000 g/mol; 58 wt.%) and polyethylene oxide (molecular mass 400 000 g/mol; 20 wt.%) containing potassium iodide of concentration 21 wt.% and 4 wt.% monosodium phosphate dihydrate. Fiber diameter 156 ± 26 nm.

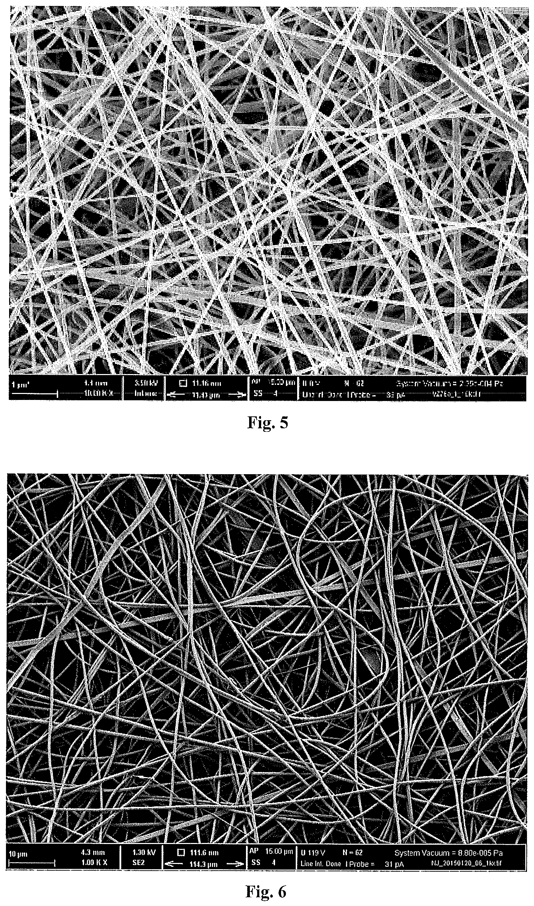

Fig. 5 displays fibrous layer of hyaluronic acid (molecular mass 137 000 g/mol; 79.83 wt.%) polyethylene oxide (molecular mass 400 000 g/mol; 3 wt.%) and polyvinyl alcohol (molecular mass 190 000 g/mol; 17 wt.%) containing octenidine of concentration 0.17 wt.%. Fiber diameter 102 ± 30 nm.
Fig. 6 displays fibrous layer of palmitoyl HA (SS 68%; molecular mass 115 kDa, 79.83 wt.%) and polyethylene oxide (molecular mass 400 000 g/mol; 20 wt.%) containing octenidine of concentration 0.17 wt.%. Fiber diameter 513 ± 114 nm.

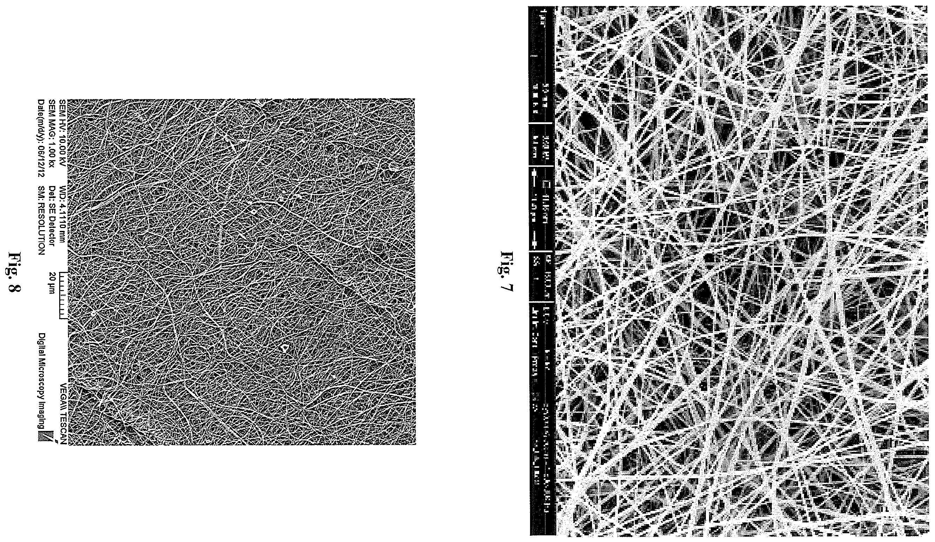

Fig. 7 displays nano-fibrous layer of metacroyl HA (SS 80%; molecular mass 93 kDa, 73 wt.%) and polyvinyl alcohol (molecular mass 190 000 g/mol; 20 wt.%) containing dexamethasone of concentration 7 wt.%.
Fig. 8 displays nano-fibrous layer of metacroyl HA (SS 80 %; molecular mass 93 kDa, 73 wt.%) and polyethylene oxide (molecular mass 600 000 g/mol; 20 wt.%) containing diclofenac of concentration 7 wt.%.

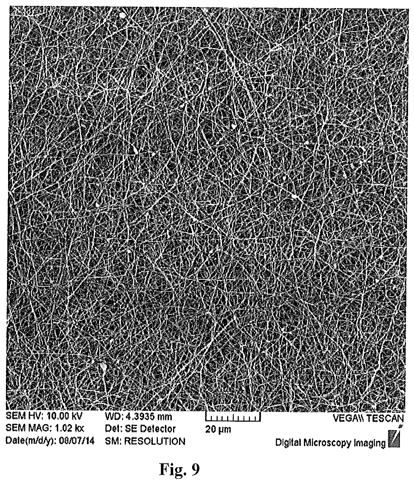

Fig. 9 displays nano-fibrous layer of furanyl HA (SS 4%; molecular mass 115 000 g/mol, 79.8 wt.%) and polyethylene oxide (molecular mass 600 000 g/mol; 20 wt.%) containing disinfectant brilliant green of concentration 0.2 wt.%.

### Examples of embodiments of the present invention

### Example 1

Octenidine hydrochloride was incorporated in the solution for the preparation of fibrous layer of hyaluronic acid and polyethylene oxide (PEO) in concentrations 0.17, 0.4 and 0.6 wt.% of dry mass. Octenidine hydrochloride was dissolved in ethyl alcohol so that the concentration is 10 wt.%. Three additional solutions were then prepared of this stock solution containing 0.17/0.4/0.6 wt.% in the order octedine hydrochloride, 20 wt.% polyethylene oxide (molecular mass 400 000 g/mol) and 79.83/79.6/79.4 wt.% in the order of hyaluronic acid (molecular mass 81 000 g/mol). The solution was filled in an injector and spun electrostatically on plate collector using the voltage 80 kV and distance 20 cm between emitter and collector. Resulting fibrous layer of areal mass 3 g/m² was cut to bands of dimensions 5x15 mm.

### Example 2

Potassium iodide was incorporated in the solution for preparation of fibrous layer of hyaluronic acid and polyethylene oxide in concentration 21 wt.% of dry mass, when additional 3 wt.% monosodium phosphate dihydrate was added. Concentration of hyaluronan (molecular mass 76 000 g/mol) made 56 wt.%. Concentration of PEO (molecular mass 400 000 g/mol) was 20 wt.% of dry mass.

The solution was filled in an injector and spun electrostatically on plate collector using voltage 60 kV and distance 20 cm between emitter and collector. Resulting fibrous layer of areal mass 5 g/m² was cut to bands of dimensions 5×15 mm.

### Example 3

Fibrous layers were prepared following the process stated in example 1, whereas the agents that were used for the preparation, are stated in Table 1 bellow:

**Table 1**

| **HA derivative** | **Degree of substitution [%]** | **Molecular mass HA [×1000 g/mol]** | **HA/HA-der mass ratio** | **Active agent** | **Active agent content [wt.%]** | **Polymer** | **molecular mass of synth. Polymer [×1000 g/mol]** | **Polymer of the mixture thereof [wt.% ]** | **Time of complete dissolution in the eye [sec]** |
|---|---|---|---|---|---|---|---|---|---|
| Furanyl | 2.2 | 81 | 1/9 | octenidine | 0.17 | PEO | 400 | 20 | 10 |
| Furanyl | 2.2 | 81 | 9/1 | Octenidine | 0.4 | PEO | 400 | 20 | 10 |
| - | - | 82.3 | 10/0 | Octenidine | 0.17 | PEO | 600 | 20 | 0.1 |
| - | - | 82.3 | 10/0 | Octenidine | 0.4 | PEO | 400 | 50 | 0.1 |
| - | - | 82.3 | 10/0 | Octenidine | 0.17 | PEO | 600 | 20 | 0.1 |
| Palmitoyl | 68 | 76 | 0/10 | Dexamethasone | 6 | PEO | 400 | 10 | 1.5 |
| Palmitoyl | 68 | 76 | 0/10 | Diclofenac | 6 | PEO | 400 | 15 | 1.5 |
| Metacroyl | 80 | 93 | 0/10 | Dexamethasone | 7 | PEO | 600 | 20 | 1 |
| Metacroyl | 80 | 93 | 0/10 | Diclofenac | 7 | PEO | 600 | 50 | 1 |
| Caproyl | 88 | 115 | 5/5 | Gentamycin | 10 | PEO | 300 | 20 | 0.5 |
| Furanyl | 4 | 76 | 5/5 | Dexamethasone | 40 | PEO | 600 | 20 | 10 |
| Palmitoyl | 75 | 68 | 2/8 | Naproxene | 50 | PEO | 400 | 15 | 1.5 |
| Caproyl | 78 | 95 | 0/10 | Octenidine | 0.17 | PVA | 190 | 20 | 0.5 |
| Caproyl | 78 | 95 | 5/5 | Dexamethasone | 6 | PVA | 190 | 20 | 0.5 |
| Caproyl | 85 | 95 | 2/8 | Diclofenac | 6 | PVA | 190 | 50 | 0.5 |
| - | - | 81 | 10/0 | Gentamycine | 10 | PEO/PVA | 400/190 | 1515 | 0.1 |
| Furanyl | 4 | 115 | 0/10 | Dexamethasone | 7 | PEO/PVA | 600/80 | 1515 | 10 |
| Palmitoyl | 65 | 137 | 515 | carbetopendecium | 5 | PEO/PVA | 400/190 | 17/3 | 1.5 |

## Claims

1. Ophthalmologic preparation containing hyaluronic acid, **characterized in that,** it is in a form of a solid fibrous carrier that comprises water soluble nano-fibers containing
- hyaluronic acid or a pharmaceutically acceptable salt thereof,
and/or at least one derivative of hyaluronic acid of a general formula I 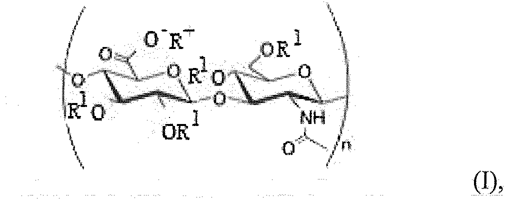
wherein R⁺ is H⁺ or pharmaceutically acceptable salt,
R¹ is H or -C(=O)CₓH_{y} or furanyl, where x is integer in the range 3 to 21 and y is integer in the range 7 to 43 and CₓH_{y} is linear or branched, saturated or unsaturated chain C₃-C₂₁, one or more R¹-C(=O)CₓH_{y} or furanyl being at least in one repeating unit and where n is in the range 12 to 4 000,
- and at least one a water soluble synthetic polymer selected from the group containing polyethylene oxide or polyvinyl alcohol, the content of the synthetic polymer or the mixture thereof being in the range 10 to 90 wt.% in the dry mass of nano-fibers contained in the ophthalmologic preparation
- and at least one active agent.

2. Ophthalmologic preparation according to claim 1, **characterized in that,** the derivative of hyaluronic acid is selected from the group containing palmitoyl hyaluronan, caproyl hyaluronan, oleyl hyaluronan, butanoyl hyaluronan, linoleoyl hyaluronan or furanyl hyaluronan.

3. Ophthalmologic preparation according to claim 1 or 2, **characterized in that,** the active agent is selected from the group containing anti-allergic, antibiotic, antimycotic, antineoplastic, anti-inflammatory, antiviral, antiglaucoma, antiseptic or diagnostic agent.

4. Ophthalmologic preparation according to claim 3, **characterized in that,** the active agent is hydrophobic, and it is contained in the nano-fibers in the form of micelles.

5. Ophthalmologic preparation according to claims 3 or 4, **characterized in that,** the content of the active agent is 0.01 to 50 wt.%, preferably 0.1 to 50 wt % in the dry mass of nano-fibers contained in the ophthalmologic preparation.

6. Ophthalmologic preparation according to any one of claims 1 to 5, **characterized in that,** the content of hyaluronic acid and/or derivative of hyaluronic acid or pharmaceutically acceptable salts thereof is in the range 5 to 90 wt. % in the dry mass of nano-fibers contained in the ophthalmologic preparation.

7. Ophthalmologic preparation according to claim 6, **characterized in that,** the mass ratio of hyaluronic acid or salt thereof and derivative of hyaluronic acid is 1/9 to 911, preferably 3/7 to 7/3.

8. Ophthalmologic preparation according to any one of the claims 1 to 7, **characterized in that,** the content of the synthetic polymer or the mixture thereof is in the range 20 to 50 wt. % in the dry mass of nano-fibers contained in the ophthalmologic preparation.

9. Ophthalmologic preparation according to the claim 8, **characterized in that,** the mass ratio of polyethylene oxide and polyvinyl alcohol in the mixture is in the range 19/1 to 3/17.

10. Ophthalmologic preparation according to any one of the claims 1 to 9, **characterized in that,** the nano-fibers have diameters in the range 50 to 1 000 nm, preferably 50 to 500 nm, more preferably 50 to 200 nm.

11. Ophthalmologic preparation according to any one of the claims 1 to 10, **characterized in that,** it is equipped with a holder for manipulation during its application.

12. Ophthalmologic preparation according to any one of claims 1 to 11 for the use in treatment or prevention of ophthalmologic states, preferably ophthalmologic diseases and disorders selected from the group comprising glaucoma, syndrome of dry eye, macular degeneration, infection, inflammation or allergy.

## Patentansprüche

1. Ein die Hyaluronsäure enthaltendes ophtalmologisches Präparat, **dadurch gekennzeichnet, dass** es in der Form eines festen faserigen Trägers vorliegt, der die folgenden Bestandteile enthaltende, wasserlösliche Nanofasern umfasst:
- Hyaluronsäure oder ein pharmazeutisch akzeptierbares Salz derselben,
und/oder wenigstens ein Derivat der Hyaluronsäure nach der allgemeinen Formel I 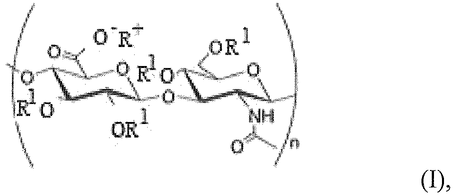
wobei R⁺ H⁺ oder ein pharmazeutisch akzeptierbares Salz ist,
R¹ für H oder -C(=O)CₓH_{y} oder Furanyl steht, wobei x eine im Bereich von 3 bis 21 liegende ganze Zahl und y eine im Bereich von 7 bis 43 liegende ganze Zahl ist und CₓH_{y} eine lineare oder verzweigte, gesättigte oder ungesättigte C₃-C₂₁-Kette ist, wobei in wenigstens einer abwechselnd vorkommenden Einheit das Glied R¹ - C(=O)CₓH_{y} oder Furanyl darstellt und wobei n in den Bereich von 12 bis 4 000 fällt,
- und wenigstens ein wasserlösliches synthetisches Polymer, das aus der Gruppe ausgewählt ist, die Polyethylenoxid oder Polyvinylalkohol umfasst, wobei der Anteil des in der Trockenmasse des nanofaserigen Bestandteils des ophthalmologischen Präparats enthaltenen synthetischen Polymers oder deren Gemisches derartiger Polymere im Bereich von 10 bis 90 Gew.-% liegt,
- sowie wenigstens eine wirksame Substanz.

2. Das ophtalmologische Präparat nach Anspruch 1, **dadurch gekennzeichnet, dass** das Derivat der Hyaluronsäure aus der Gruppe ausgewählt ist, die Palmitoyl-Hyaluronan, Caproyl-Hyaluronan, Oleyl-Hyaluronan, Butanoyl-Hyaluronan, Linoleoyl-Haluronan oder Furanyl- Hyaluronan umfasst.

3. Das ophtalmologische Präparat nach Anspruch 1 oder 2, **dadurch gekennzeichnet, dass** die wirksame Substanz aus der Gruppe ausgewählt ist, die antiallergische, antibiotische, antimykotische, antineoplastische, entzündungshemmende, antivirale, antiglaukomatöse, antiseptische oder diagnostische Substanzen umfasst.

4. Das ophtalmologische Präparat nach Anspruch 3, **dadurch gekennzeichnet, dass** die wirksame Substanz hydrophobisch ist und in den Nanofasern in der Form von Mizellen enthalten ist.

5. Das ophtalmologische Präparat nach Anspruch 3 oder 4, **dadurch gekennzeichnet, dass** der Anteil der in der Trockenmasse des nanofaserigen Bestandteils des ophthalmologischen Präparats enthaltenen wirksamen Substanz im Bereich von 0,01 bis 50 Gew.-%, vorzugsweise im Bereich von 0,1 bis 50 Gew.-%, liegt.

6. Das ophtalmologische Präparat nach einem der Ansprüche 1 bis 5, **dadurch gekennzeichnet, dass** der Anteil der in der Trockenmasse des nanofaserigen Bestandteils des ophthalmologischen Präparats enthaltenen Hyaluronsäure und/oder deren Derivats oder der pharmazeutisch akzeptierbaren Salze derselben im Bereich von 5 bis 90 Gew.-% liegt.

7. Das ophtalmologische Präparat nach Anspruch 6, **dadurch gekennzeichnet, dass** das Gewichtsverhältnis zwischen der Hyaluronsäure oder einem Salz derselben und dem Derivat der Hyaluronsäure im Bereich von 1/9 bis 9/1, vorzugsweise im Bereich von 3/7 bis 7/3, liegt.

8. Das ophtalmologische Präparat nach einem der Ansprüche 1 bis 7, **dadurch gekennzeichnet, dass** der Anteil des in der Trockenmasse des nanofaserigen Bestandteils des ophthalmologischen Präparats enthaltenen synthetischen Polymers oder des Gemisches derartiger Polymere im Bereich von 20 bis 50 Gew.-% liegt.

9. Das ophtalmologische Präparat nach Anspruch 8, **dadurch gekennzeichnet, dass** das Gewichtsverhältnis zwischen Polyethylenoxid und Polyvinylalkohol in dem Gemisch 19/1 bis 3/17 beträgt.

10. Das ophtalmologische Präparat nach einem der Ansprüche 1 bis 9, **dadurch gekennzeichnet, dass** der Durchmesser der Nanofasern im Bereich von 50 bis 1 000 nm, vorzugsweise von 50 bis 500 nm, vorzüglicherweise von 50 bis 200 nm liegt.

11. Das ophtalmologische Präparat nach einem der Ansprüche 1 bis 10, **dadurch gekennzeichnet, dass** es mit einer Halterung ausgestattet ist, die zur Handhabung während der Anwendung des Präparats vorgesehen ist.

12. Das ophtalmologische Präparat nach einem der Ansprüche 1 bis 11, zur Anwendung bei der Behandlung oder Vorbeugung von ophthalmologischen Zuständen, vorzugsweise ophthalmologischen Erkrankungen oder Störungen, die aus der Gruppe ausgewählt sind, die Glaukom, Trockenauge-Syndrom, Makuladegeneration, Infektion, Entzündung oder Allergie umfasst.

## Revendications

1. Préparation ophtalmologique contenant de l'acide hyaluronique, **caractérisée en ce qu'**elle est en forme d'un support fibreux solide qui comprend des nanofibres hydrosolubles contenant
- de l'acide hyaluronique ou un sel pharmaceutiquement acceptable de celle-ci, et/ou au moins un dérivé de l'acide hyaluronique selon la formule générale I 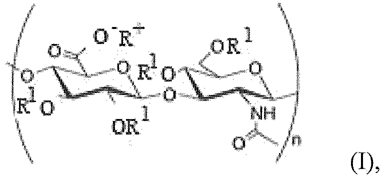
où R⁺ est H⁺ ou un sel pharmaceutiquement acceptable,
R¹ est H ou -C(=O)CₓH_{y} ou furanyle, où x est un nombre entier dans la plage de 3 à 21 et y est un nombre entier dans la plage de 7 à 43 et CₓH_{y} est une chaine C₃-C₂₁ linéaire ou ramifiée, saturée ou insaturée, un ou plusieurs R¹-C(=O)CₓH_{y} ou furanyle étant dans au moins un motif répété et où n est dans la plage de 12 à 4000,
- et au moins un polymère synthétique hydrosoluble sélectionné dans le groupe contenant un oxyde de polyéthylène ou un alcool polyvinylique, la teneur en polymère synthétique ou en mélange de ceux-ci étant dans la plage de 10 à 90 % en poids dans la masse sèche de nanofibres dans la préparation ophtalmologique,
- et au moins un agent actif.

2. Préparation ophtalmologique selon la revendication 1, **caractérisée en ce que** le dérivé de l'acide hyaluronique est sélectionné dans le groupe contenant palmitoyle hyaluronane, caproyle hyaluronane, oleyle hyaluronane, butanoyle hyaluronane, linoleoyle hyaluronane ou furanyle hyluronane.

3. Préparation ophtalmologique selon la revendication 1 ou 2, **caractérisée en ce que** l'agent actif est sélectionné dans le groupe contenant un agent anti-allergique, antibiotique, antimycotique, antinéoplasique, anti-inflammatoire, antiviral, antiglaucome, antiseptique ou diagnostique.

4. Préparation ophtalmologique selon la revendication 3, **caractérisée en ce que** l'agent actif est hydrophobique et **en ce qu'**il est contenu dans les nanofibres sous la forme de micelles.

5. Préparation ophtalmologique selon la revendication 3 ou 4, **caractérisé en ce que** le contenu de l'agent actif est de 0.01 à 50 % en poids, préférablement de 0.1 à 50 % en poids dans la masse sèche des nanofibres contenus dans la préparation ophtalmologique.

6. Préparation ophtalmologique selon l'une quelconque des revendications 1 à 5, **caractérisée en ce que** la teneur en acide hyaluronique et/ou en dérivé de l'acide hyaluronique ou en sels pharmaceutiquement acceptables de ceux-ci est dans la plage de 5 à 90 % en poids dans la masse sèche des nanofibres contenus dans la préparation ophtalmologique.

7. Préparation ophtalmologique selon la revendication 6, **caractérisée en ce que** le rapport de masse de l'acide hyaluronique ou d'un sel de celle-ci au dérivé de l'acide hyaluronique est de 1/9 à 9/1, préférablement de 3/7 à 7/3.

8. Préparation ophtalmologique selon l'une quelconque des revendications 1 à 7, **caractérisée en ce que** la teneur en polymère synthétique ou en mélange de celui-ci est dans la plage de 20 à 50 % en poids dans la masse sèche des nanofibres contenus dans la préparation ophtalmologique.

9. Préparation ophtalmologie selon la revendication 8, **caractérisée en ce que** le rapport de masse de l'oxyde de polyéthylène au polyvinyle alcool dans le mélange est dans la plage de 19/1 à 3/17.

10. Préparation ophtalmologique selon l'une quelconque des revendications 1 à 9, **caractérisée en ce que** les nanofibres ont des diamètres dans la plage de 50 à 1000 nm, préférablement de 50 à 500 nm, plus préférablement de 50 à 200 nm.

11. Préparation ophtalmologique selon l'une quelconque des revendications 1 à 10, **caractérisée en ce qu'**elle est pourvue d'un support pour la manipulation pendant son application.

12. Préparation ophtalmologique selon l'une quelconque des revendications 1 à 11 pour utilisation dans le traitement ou dans la prévention d'états ophtalmologiques, préférablement de maladies et troubles ophtalmologiques sélectionnés dans le groupe comprenant le glaucome, le syndrome de l'œil sec, la dégénérescence maculaire, l'infection, l'inflammation ou l'allergie.
